# EUROPÄISCHE PATENTSCHRIFT

(11) **EP 0 885 898 B1**
(45) Veröffentlichungstag und Bekanntmachung des Hinweises auf die Patenterteilung: **21.01.2004**
(21) Anmeldenummer: 98110116.5
(22) Anmeldetag: 03.06.1998
(51) Int. Cl.: C07H 13/06

(54) **Verfahren zur Herstellung von Saccharosefettsäureestern**
Method of preparation of saccharose fatty acid esters
Méthode pour la préparation d'esters gras de saccharose

(30) Priorität: 17.06.1997 DE 19725548
(43) Veröffentlichungstag der Anmeldung: 23.12.1998
(73) Patentinhaber: Goldschmidt AG, 45127 Essen (DE)
(72) Erfinder: Desai, Natvarlal, Dr., 46537 Dinslaken (DE); Grüning, Burghard, Dr., 45134 Essen (DE)

(56) Entgegenhaltungen:
- DD-A- 227 137
- DE-A- 4 212 155
- US-A- 4 377 685
- US-A- 4 927 920

## Beschreibung

Gegenstand der Erfindung ist ein Verfahren zur lösungsmittelfreien Herstellung von Saccharosefettsäureestern, deren Gemischen mit Nichtzuckerpolyolfettsäureestern und insbesondere von Saccharoseglyceriden.

Saccharosefettsäureester sind seit Jahrzehnten Gegenstand intensiver Untersuchungen, da sie aus nachwachsenden, nicht erschöpfenden Naturstoffen, wie Zucker und Fetten, hergestellt werden können. Beide Substanzgruppen werden als wertvolle, milde, physiologisch unbedenkliche und biologisch abbaubare Zusätze in der Kosmetik, Pharmazie, in Lebensmitteln, in Futtermitteln sowie als Agrar-Chemikalien, zum Beispiel zur Obstfrischhaltung, eingesetzt.

Saccharoseglyceride stellen eine besondere Form der Bereitstellung von Saccharosefettsäureestern dar. Sie sind im allgemeinen Gemische aus Saccharoseestern und Glyceriden. Saccharoseglyceride, die für den Nahrungsmittelbereich zugelassen sind, müssen gemäß der europäischen Norm E474 wenigstens 40 % Saccharoseester und wenigstens 40 % Glyceride enthalten.

Im Stand der Technik ist eine Reihe von Verfahren zur Herstellung von Saccharoseestern bekannt. So wird die Umsetzung von Fettsäuremethylestern mit Saccharose in Gegenwart von basischen Katalysatoren in verschiedenen Lösungsmitteln, wie Dimethylformamid oder Dimethylsulfoxid als Lösungsmittel, beschrieben, siehe beispielsweise die DE-C-10 52 388 oder die DE-C-12 62 988.

Ein großer Nachteil dieses Verfahrens besteht darin, daß die Lösungsmittel Dimethylformamid (DMF) oder Dimethylsulfoxid (DMSO) nach der Umesterung restlos aus dem Reaktionsgemisch entfernt werden müssen, insbesondere wenn die Produkte im Lebensmittelbereich eingesetzt werden sollen.

Saccharoseglyceride können prinzipiell über die Umesterung von natürlichen Fetten oder pflanzlichen Ölen mit Saccharose hergestellt werden. Eine wirtschaftliche Herstellung von Saccharoseglyceriden über die Umesterung in industriellem Maßstab ist jedoch kaum realisierbar, da das entstehende Glycerin wegen seines hohen Siedepunktes nur schwer zu entfernen ist. Darüber hinaus bereiten die thermische Instabilität und die relativ begrenzte Löslichkeit der Saccharose in organischen Lösungsmitteln erhebliche Probleme. Daher müssen die Lösungsmittel, beispielsweise DMF, DMSO, N-Methylpyrrolidon (NMP) oder Pyridin, in großem Überschuß zugesetzt und dennoch nach der Reaktion restlos entfernt werden. So wird in der DE-C-11 93 026 ein Verfahren zur Herstellung von Polyfettsäureestern nicht reduzierender Oligosaccharide durch Umestern eines nichtreduzierenden Oligosaccharids mit Fettsäureestern von Nichtzuckeralkoholen beschrieben, wobei die Reaktion in Gegenwart eines Umesterungskatalysators bei Anwesenheit von Pyridin als Reaktionsmedium durchgeführt wird.

In ähnlicher Weise beschreibt die US-A-3 349 081 ein Verfahren zur Herstellung von Saccharoseestern durch Umesterung von Saccharose mit natürlichen Triglyceriden in Dimethylformamid, gefolgt vom Abziehen des Reaktionslösungsmittels und Behandeln des Rückstands mit einer wäßrig/butanolischen Natriumchloridlösung und Verdampfen der separierten Butanolschicht bis zur Trockne.

Weiterhin ist aus L. Bobichon, ACS Sym. Ser (1977), 41, Sucrochemistry Sympo. 1976, S. 115 bis 120 die Herstellung von Saccharoseglyceriden aus Talg beziehungsweise Palmkernöl und Saccharose in Dimethylformamid bekannt. Die Produkte, CELINOLE®, enthalten 39 bis 42 % Saccharoseester, 50 bis 55 % Glyceride und 50 bis 100 ppm DMF und werden als Futtermittelzusätze empfohlen.

In neuerer Zeit wurden Versuche unternommen, die durch den Lösungsmitteleinsatz bedingten Nachteile des Standes der Technik zu vermeiden. Aus der GB-A-1 399 053 ist ein lösungsmittelfreies Umesterungsverfahren zur Herstellung von Saccharoseestern bekannt. Nach diesem Verfahren wird die Umesterung von Fettsäureglyceriden mit Saccharose insbesondere unter Einwirkung von Kaliumcarbonat in einer Menge von 5 bis 12 %, bezogen auf das Gesamtgewicht der Reaktionsmischung, bei 110 bis 140 °C durchgeführt. Die nach diesem Verfahren erhaltenen Produkte sind sehr dunkel, insbesondere braune wachsartige Materialien. Darüber hinaus liegt die Gesamtausbeute an Mono- und Disaccharoseestern bei deutlich unter 30 %. Dieses Verfahren liefert ein komplexes Reaktionsgemisch, das in erhöhtem Maße nicht reagierte Ausgangsprodukte enthält.

In der GB-A-1 499 989 wird ebenfalls die Umsetzung von fester Saccharose mit einem Alkylester einer Fettsäure mit 1 bis 6 C-Atomen in der Alkoholeinheit und wenigstens 8 Kohlenstoffatomen in der Fettsäureeinheit beschrieben, wobei in Anwesenheit von basischen Umesterungskatalysatoren bei einer Temperatur von 110 bis 140 °C unter Atmosphärendruck in Abwesenheit von Lösungsmitteln umgesetzt wird. Auch hier wird, wie den Ausführungsbeispielen zu entnehmen ist, Kaliumcarbonat besonders bevorzugt als Katalysator eingesetzt. Die beiden vorgenannten Verfahren liefern trotz der hohen Einsatzmenge an basischem Katalysator und sehr langer Reaktionszeit von 6 bis 23 Stunden sehr niedrige Ausbeuten an Saccharoseestern. Darüber hinaus enthalten die Reaktionsgemische in erheblichem Umfang nicht reagierte Alkylester, Glyceride, Seifen und Saccharose. Derartige Reaktionsgemische können daher nach der dort beschriebenen Produktzusammensetzung kaum für Lebensmittelanwendungen geeignet sein oder gar die Anforderungen an die EG-Norm E474 für Lebensmittelemulgatoren erfüllen.

Aus der FR-A-2 463 152 ist ein weiteres Verfahren zur Herstellung von Saccharoseglyceriden bekannt, bei dem in der ersten Reaktionsstufe eine begrenzte Alkoholyse des Triglycerids mit Alkohol unter Zusatz von basischem Katalysator Kaliumcarbonat bei 80 bis 180 °C erfolgt. In der zweiten Reaktionsstufe wird das Gemisch aus Alkylester, Triglycerid und Seife mit Saccharose unter erneuter Zugabe von Kaliumcarbonat umgeestert. Das Verfahren umfaßt somit zwei Reaktionsschritte, erfordert hohe Einsatzmengen an Katalysator und Seifen und liefert dabei jedoch ein Reaktionsgemisch aus Saccharoseestern (40 bis 49 %), Glyceriden und hohen Mengen an unerwünschten, nicht reagierten Alkylestern (2 bis 20 %) sowie Seife und Saccharose.

Aus EP-A-0 404 226 ist ein Verfahren zur Reinigung von Produkten enthaltend Ester von nicht reduzierenden Zuckern und eine oder mehrere Fettsäuren bekannt. Hierbei wird in einem aufwendigen Verfahren das rohe Veresterungsprodukt einer Extraktion mit überkritischem Kohlendioxid unterzogen.

Die DE-A-41 31 505 betrifft ein Verfahren zur Aufarbeitung des bei der lösungsmittelfreien Herstellung von Saccharosefettsäureestern durch Umesterung von Saccharose mit Fettsäurealkylester, insbesondere Fettsäuremethylester, in Gegenwart eines basischen Umesterungskatalysators erhaltenen Reaktionsgemisches. Um die Wirtschaftlichkeit des Verfahrens bei gleicher oder sogar gesteigerter Qualität des Endprodukts erheblich zu verbessern, wird vorgeschlagen, daß nicht umgesetzte Saccharose bei einer Temperatur zwischen den Schmelzpunkten der eingesetzten Saccharose und des hergestellten Saccharoseesters abfiltriert und danach nicht umgesetzte Alkylester aus dem Reaktionsgemisch abdestilliert werden.

Gegenstand der DE 42 12 155 ist ein Verfahren zur Herstellung von Polyol-Fettsäure-Partialestern mit einem hohen Anteil an Monoester, gemäß dem
1) man in einer ersten Stufe Saccharose mit überschüssigem Fettsäureester umsetzt; und
2) das erhaltene Produkt in einer zweiten Stufe mit einem einwertigen Alkohol mit 1 bis 8 C-Atomen umsetzt; und
3) das Rohprodukt anschliessend von gebildetem Fettsäuremethylester destillativ befreit.

Die Aufgabe der vorliegenden Erfindung bestand darin, ein Verfahren zur lösungsmittelfreien Herstellung von Saccharosefettsäureestern - verkürzt auch Saccharoseester genannt -, deren Gemischen mit Nichtzuckerpolyolfettsäureestern insbesondere von Saccharoseglyceriden zur Verfügung zu stellen, welches in einfacher Weise durchführbar ist, sich besonders für die Anwendung in industriellem Maßstab eignet und gleichzeitig die durch Verwendung toxischer Lösungsmittel sowie durch aufwendige und kostspielige Reinigungsschritte auftretenden Probleme des Standes der Technik vermeidet.

Das Verfahren sollte zweckmäßigerweise Saccharoseester in hohen Ausbeuten ergeben, aus denen sich gegebenenfalls durch einfaches Beimischen von Glyceriden EG-Norm E474-konforme Saccharoseglyceride realisieren lassen.

Die vorgenannte Aufgabe wird erfindungsgemäß in einer ersten Ausführungsform gelöst durch ein Verfahren zur lösungsmittelfreien Herstellung von Saccharosefettsäureestern, wobei man
a) Saccharose mit Fettsäurealkylestern einer Kettenlänge von 6 bis 20 C-Atomen im geradkettigen oder verzweigten, gesättigten, einfach oder mehrfach ungesättigten Fettsäurerest und 1 bis 6 C-Atomen im geradkettigen oder verzweigten Alkoholrest in Anwesenheit basischer Katalysatoren bei einem Druck von 25 bis 100 mbar und einer Temperatur von 120 bis 160 °C umsetzt,
   dadurch gekennzeichnet, daß man
b) das Reaktionsgemisch gemäß a) mit 0,01 bis 0,5 Mol Polyol pro Mol eingesetzten Fettsäurealkylesters bei einem Druck von 25 bis 100 mbar und einer Temperatur im Bereich von 90 bis 130 °C im Verlauf von 0,5 bis 3 Stunden umsetzt und anschließend
c) ohne Zusatz von Lösungsmittel filtriert.

Der Vorteil des erfindungsgemäßen Verfahrens besteht insbesondere darin, auf sehr einfache Weise die Nachteile des Standes der Technik bedingt durch die Reaktionsführung in toxischen Lösungsmitteln sowie die aufwendige und kostspielige Extraktion in organischen Lösungsmitteln oder in überkritischen Lösungsmitteln wie Kohlendioxid sowie die Molekulardestillation des nicht reagierten Alkylesters zu vermeiden. Das erfindungsgemäße Verfahren bietet daher eine hohe Wirtschaftlichkeit.

Die Saccharosefettsäureester des erfindungsgemäßen Verfahrens weisen sehr hohe Saccharoseestergehalte auf und sind ferner frei von unerwünschtem, nicht reagiertem Alkylester. Der Gehalt an nicht reagierter Saccharose ist in den erfindungsgemäßen Produkten sehr niedrig.

Es ist mit Hilfe der Erfindung ohne weiteres möglich, Saccharoseglyceride herzustellen, die mehr als 80 Gew.-% Saccharoseester enthalten und somit auch die Spezifikation der EG-Norm E473 (Saccharoseester) erfüllen und daher für den Lebensmittelbereich zusätzlich wertvoll und interessant sind.

Als Fettsäurealkylester zur Durchführung des erfindungsgemäßen Verfahrens kommen sowohl geradkettige oder verzweigte, gesättigte, einfach oder mehrfach ungesättigte Fettsäurealkylester mit einer Kettenlänge von 6 bis 20 C-Atomen im Fettsäurerest in Frage. Besonders bevorzugt im Sinne der vorliegenden Erfindung sind Fettsäurealkylester mit einer Kettenlänge von 12 bis 18 C-Atomen im Fettsäurerest. Die Auswahl der Alkoholgruppen der Fettsäurealkylester ist prinzipiell nicht eingeschränkt. Besonders bevorzugt im Sinne der vorliegenden Erfindung wird jedoch der Methylester eingesetzt. Anstelle der reinen Fettsäurealkylester mit definierter Kettenlänge des Fettsäurerestes können selbstverständlich auch übliche Fettsäurealkylestergemische eingesetzt werden.

Als Polyole sind solche Nichtzuckeralkohole geeignet, die mehr als eine Hydroxylgruppe aufweisen. Geeignete Polyole sind z. B. 1,2-Propylenglykol, Glycerin, Sorbitol sowie Kondensationsprodukte des Glycerins, wie Di-, Tri- oder Tetraglycerin oder deren Gemische. Ein besonders bevorzugtes Polyol ist Glycerin. Wird Glycerin eingesetzt, entstehen Saccharoseglyceride.

Zu den für das Verfahren der Erfindung geeigneten alkalischen Katalysatoren zählen verschiedene anorganische Salze, wie Oxide, Carbonate, Hydroxide, Hydrogencarbonate sowie Kalium-, Natrium-, Magnesium-, Zink- und Lithiumseifen der Fettsäuren mit einer Kettenlänge von 8 bis 20 C-Atomen in geradkettigen oder verzweigten, gesättigten, einfach oder mehrfach ungesättigten Fettsäureresten. Es können die anorganischen Salze allein oder auch in Kombination mit den genannten Seifen verwendet werden. Im Sinne der vorliegenden Erfindung besonders bevorzugt ist die Anwesenheit von 0,5 bis 5 Gew.-% des Katalysators, bezogen auf das Gesamtgewicht des Reaktionsgemisches.

Für die Durchführung des erfindungsgemäßen Verfahrens werden die einzelnen Komponenten eingewogen. Die Umesterung wird vorzugsweise bei einer Temperatur im Bereich von 120 bis 160 °C, insbesondere bei 120 bis 145 °C im Vakuum, das heißt bei vermindertem Druck, der vorzugsweise 25 bis 100 mbar beträgt, insbesondere im Verlauf von 2 bis 6 Stunden durchgeführt. Hierbei kann ein äquimolares Stoffmengenverhältnis von Fettsäurealkylester zu Saccharose eingestellt werden. Darüber hinaus ist es jedoch bevorzugt, einen Überschuß von Fettsäurealkylester, insbesondere Fettsäuremethylester, zu Saccharose einzusetzen. Besonders bevorzugt im Sinne der vorliegenden Erfindung wird dieser Stoffmengenbereich im Verhältnis von 1 bis 3,5 Mol Fettsäurealkylester zu 1 Mol Saccharose eingestellt.

Im Anschluß an die Veresterung der Saccharose wird die Reaktionsmasse gegebenenfalls abgekühlt und mit 0,01 bis 0,5 Mol Polyol pro Mol eingesetzten Fettsäurealkylesters bei reduziertem Druck und erhöhter Temperatur umgesetzt. Besonders bevorzugt im Sinne der vorliegenden Erfindung ist es, die Reaktion bei 90 bis 130 °C, einem Druck von 25 bis 100 mbar im Verlauf von 0,5 bis 3 Stunden durchzuführen.

Überraschenderweise wurde gefunden, daß nach dem erfindungsgemäßen Verfahren bevorzugt nicht reagierte Fettsäurealkylester in Polyolester, insbesondere in Glyceride umgeestert werden und hierbei keine nennenswerte Umesterung vom Saccharoseester stattfindet.

Die erfindungsgemäß erhaltenen Reaktionsprodukte können bei Bedarf nach an sich bekannten Verfahren, die beispielsweise in der DE-A-41 31 505 offenbart sind, mit anorganischen oder organischen Säuren neutralisiert werden und/oder mit Wasserstoffperoxid bei erhöhter Temperatur, beispielsweise bei 80 °C, im Verlauf von 30 min gebleicht werden. Besonders bevorzugt im Sinne der vorliegenden Erfindung werden die Saccharoseester im Gemisch mit Fettsäurepolyolester, insbesondere Saccharoseglycerid, als rohes Reaktionsprodukt bei 80 bis 100 °C, gegebenenfalls unter Zusatz von Filterhilfsmittel und 0,5 bis 2 bar ohne Zusatz von organischen oder anorganischen Lösungsmitteln filtriert.

Falls gewünscht, werden die viskosen Reaktionsprodukte bei 60 bis 70 °C mit Wasser versetzt, um sie in pastöser Form zu erhalten oder, je nach Anwendungsbedarf mit Glyceriden, anderen Wirkstoffen und/oder Emulgatoren versetzt und pelletiert. Die nach dem erfindungsgemäßen Verfahren erhältlichen Produkte sind hell bis cremefarbig, geruchlos und können ohne weitere Reinigung in der Kosmetik, der Pharmazie oder, sofern Glycerin als Polyol verwendet wird, in Lebensmitteln eingesetzt werden.

In den folgenden Ausführungsbeispielen wird die Erfindung näher erläutert.

### Beispiel 1

In einem Dreihalskolben mit vakuumdichtem Rührwerk, regelbarer Temperaturmeßeinrichtung und absteigendem Kühler mit kühlbarer Vorlage werden 207 g (0,965 Mol) Laurinsäuremethylester mit 102 g (0,299 Mol) Saccharose, 3 g Kaliumcarbonat und 3 g Natriumstearat bei 135 °C und 100 mbar Vakuum innerhalb von 5 Stunden umgeestert. Hierbei entsteht eine dunkelbraune viskose Masse mit der Zusammensetzung A (siehe nachfolgende Tabelle 1). Die Reaktionsmasse wird auf 90 °C abgekühlt und anschließend mit 5 g Glycerin bei 90 °C und 50 mbar Vakuum in 1,5 Stunden umgesetzt. Danach wird das Produkt mit 2,6 g Essigsäure neutralisiert und mit 1,5 g H₂O₂ (50 %ig) bei 90 °C in 30 Minuten gebleicht. Die Reaktionsmischung wird zum Schluß unter Zugabe von Filterhilfsmittel, CELITE®, 2 g, ohne Lösungsmittel, bei 90 °C und 0,5 bar Druck filtriert. Hierbei wird eine hellgelbe viskose Masse mit der Zusammensetzung B (siehe nachfolgende Tabelle 1) erhalten.

Die Zusammensetzung wird jeweils gaschromatographisch bestimmt.

**Tabelle 1**

| Produktzusammensetzung | Produkt A Gew.-% | Produkt B Gew.-% |
|---|---|---|
| Glycerin | - | <0,5 |
| SE | 82, 0 | 79, 5 |
| Glyceride | - | 7,5 |
| FSME | 2, 9 | <0, 5 |
| FS | 8, 5 | 7, 5 |
| Saccharose | 5, 0 | 3, 3 |
| n. ident. | 1,6 | 1,3 |
| SE | 82, 0 | - |
| SGL | - | 87,0 |
| SE: Saccharoseester | | |
| FSME: Fettsäuremethylester | | |
| FS: Fettsäure | | |
| SGL: Saccharoseglycerid | | |

### Beispiel 2

Eine Mischung aus 283,5 g (1,17 Mol) Myristinsäuremethylester, 145,39 g(0,43 Mol) Saccharose, 5 g Kaliumcarbonat und 5 g Zinkstearat wird wie in Beispiel 1 in 5 Stunden umgesetzt. Das Reaktionsprodukt A (siehe Tabelle 2) wird anschließend mit 17,5 g Glycerin bei 90 °C und 50 mbar Vakuum versetzt und unter Zugabe von 2 g Filterhilfsmittel Kieselgur (Seitz Ultra) filtriert, wobei ein helles, cremefarbenes Produkt mit der Zusammensetzung B (siehe Tabelle 2) erhalten wird.

Die Zusammensetzung wird jeweils gaschromatographisch bestimmt.

**Tabelle 2**

| Produktzusammensetzung | Produkt A Gew.-% | Produkt B Gew.-% |
|---|---|---|
| Glycerin | - | <0,5 |
| SE | 81, 0 | 67,0 |
| Glyceride | - | 17,5 |
| FSME | 3, 7 | <0, 5 |
| FS | 6, 9 | 8, 5 |
| Saccharose | 5, 0 | 2, 5 |
| n. ident. | 3, 4 | 3, 5 |
| SE | 81, 0 | - |
| SGL | - | 84, 5 |

### Beispiel 3

Eine Mischung aus 190,8 g (0,89 Mol) Laurinsäuremethylester, 94,57 g (0,33 Mol) Palmitin/Stearinsäuremethylester, 4 g Kaliumcarbonat und 4 g Magnesiumstearat wird wie in Beispiel 1 mit 152,38 g (0,445 Mol) Saccharose in 5 Stunden umgesetzt. Man erhält das Produkt A (siehe Tabelle 3). Das Produkt wird mit 10 g Glycerin bei 90 °C und 50 mbar Vakuum wie in Beispiel 1 behandelt, gebleicht und unter Zugabe von 2 g Filterhilfsmittel filtriert, wobei ein helles Produkt mit der Zusammensetzung B (siehe Tabelle 3) erhalten wird.

Die Zusammensetzung wird jeweils gaschromatographisch bestimmt.

**Tabelle 3**

| Produktzusammensetzung | Produkt A Gew.-% | Produkt B Gew.-% |
|---|---|---|
| Glycerin | - | <0,50 |
| SE | 81, 50 | 74, 50 |
| Glyceride | - | 6,50 |
| FSME | 2, 69 | <0,5 |
| FS | 9, 00 | 8, 50 |
| Saccharose | 5, 20 | 3, 0 |
| n . ident. | 1,61 | 6, 50 |
| SE | 81, 50 | - |
| SGL | - | 81,00 |

### Beispiel 4

Eine Mischung aus 212 g (0,786 Mol) Palmitinsäuremethylester, 57,37 g (0,27 Mol) Laurinsäuremethylester, 5 g Kaliumoxid, 5 g Natriumstearat und 122,5 g Saccharose (0,36 Mol) wird wie in Beispiel 1 umgesetzt. Das Reaktionsprodukt (A) (siehe Tabelle 4) wird anschließend mit 15 g Glycerin umgesetzt, gebleicht und filtriert (B) (siehe Tabelle 4).

Die Zusammensetzung wird jeweils gaschromatographisch bestimmt.

**Tabelle 4**

| Produktzusammensetzung | Produkt A Gew.-% | Produkt B Gew.-% |
|---|---|---|
| Glycerin | - | <0,50 |
| SE | 80, 00 | 74, 00 |
| Glyceride | - | 12,50 |
| FSME | 8,15 | <0, 5 |
| FS | 8, 00 | 7, 50 |
| Saccharose | 3,00 | 1,50 |
| n. ident. | 0,84 | 3,50 |
| SE | 80,00 | - |
| SGL | - | 86, 50 |

## Patentansprüche

1. Verfahren zur lösungsmittelfreien Herstellung von Saccharosefettsäureestern, wobei man
a) Saccharose mit Fettsäurealkylestern einer Kettenlänge von 6 bis 20 C-Atomen im geradkettigen oder verzweigten, gesättigten, einfach oder mehrfach ungesättigten Fettsäurerest und 1 bis 6 C-Atomen im geradkettigen oder verzweigten Alkoholrest in Anwesenheit basischer Katalysatoren bei einem Druck von 25 bis 100 mbar und einer Temperatur von 120 bis 160 °C umsetzt,
**dadurch gekennzeichnet, daß** man
b) das Reaktionsgemisch gemäß a) mit 0,01 bis 0,5 Mol Polyol pro Mol eingesetzten Fettsäurealkylesters bei einem Druck von 25 bis 100 mbar und einer Temperatur von 90 bis 130 °C im Verlauf von 0,5 bis 3 Stunden umsetzt und anschließend
c) ohne Zusatz von Lösungsmitteln filtriert.

2. Verfahren nach Anspruch 1, **dadurch gekennzeichnet, daß** man als Polyol Glycerin verwendet.

3. Verfahren nach Anspruch 1, **dadurch gekennzeichnet, daß** man Fettsäurealkylester mit einer Kettenlänge von 12 bis 18 C-Atomen im Fettsäurerest einsetzt.

4. Verfahren nach Anspruch 1, **dadurch gekennzeichnet, daß** man Fettsäurealkylester mit einer Kettenlänge von 1 bis 6 C-Atomen im geradkettigen oder verzweigten Alkoholrest einsetzt.

5. Verfahren nach Anspruch 1, **dadurch gekennzeichnet, daß** man Fettsäuremethylester im Verfahrensschritt a) einsetzt.

6. Verfahren nach Anspruch 1, **dadurch gekennzeichnet, daß** die Reaktion gemäß Verfahrensschritt a) die Anwesenheit von 0,5 bis 5 Gew.-% des Katalysators, bezogen auf das Gesamtgewicht des Reaktionsgemisches, umfaßt.

7. Verfahren nach Anspruch 6, **dadurch gekennzeichnet, daß** der Katalysator ausgewählt ist aus der Gruppe umfassend Alkalimetallhydroxide, insbesondere Natriumhydroxid und Kaliumhydroxid, Alkalimetallcarbonate, insbesondere Kaliumcarbonat und Cäsiumcarbonat, Seifen und Metallseifen der Fettsäuren von Natrium, Kalium, Magnesium, Zink, Zinn und/oder Lithium mit einer Kettenlänge von 6 bis 20 C-Atomen in geradkettigen oder verzweigten, gesättigten, einfach oder mehrfach ungesättigten Fettsäureresten.

8. Verfahren nach Anspruch 1, **dadurch gekennzeichnet, daß** die Reaktion gemäß Verfahrensschritt a) mit einem äquimolaren Stoffmengenverhältnis von Fettsäurealkylester zu Saccharose oder einem Überschuß von Fettsäurealkylester, insbesondere in einem Stoffmengenverhältnis von Fettsäurealkylester zu Saccharose im Bereich von 1 zu 1 bis 3, 5 zu 1 durchgeführt wird.

9. Verfahren nach Anspruch 1, **dadurch gekennzeichnet, daß** man die Filtration gemäß Verfahrensschritt c) bei 80 bis 100 °C und einem Druck von 0,5 bis 2 bar, gegebenenfalls in Anwesenheit von 0,5 bis 2 Gew.-% Filterhilfsmittel durchführt.

## Claims

1. Process for the solvent-free preparation of sucrose fatty acid esters, in which
a) sucrose is reacted with fatty acid alkyl esters of a chain length of 6 to 20 C atoms in the straight-chain or branched, saturated, mono- or polyunsaturated fatty acid radical and 1 to 6 C atoms in the straight-chain or branched alcohol radical in the presence of basic catalysts at a pressure from 25 to 100 mbar and a temperature from 120 to 160°C,
**characterized in that**
b) the reaction mixture according to a) is reacted at a pressure from 25 to 100 mbar and a temperature from 90 to 130°C in the course of 0.5 to 3 hours with 0.01 to 0.5 mol of polyol per mole of fatty acid alkyl ester employed and then
c) filtered without addition of solvent.

2. Process according to Claim 1, **characterized in that** the polyol used is glycerol.

3. Process according to Claim 1, **characterized in that** fatty acid alkyl esters having a chain length of 12 to 18 C atoms in the fatty acid radical are employed.

4. Process according to Claim 1, **characterized in that** fatty acid alkyl esters having a chain length of 1 to 6 C atoms in the straight-chain or branched alcohol radical are employed.

5. Process according to Claim 1, **characterized in that** fatty acid methyl esters are employed in process step a) .

6. Process according to Claim 1, **characterized in that** the reaction according to process step a) comprises the presence of 0.5 to 5% by weight of the catalyst, based on the total weight of the reaction mixture.

7. Process according to Claim 6, **characterized in that** the catalyst is selected from the group comprising alkali metal hydroxides, in particular sodium hydroxide and potassium hydroxide, alkali metal carbonates, in particular potassium carbonate and cesium carbonate, soaps and metal soaps of the fatty acids of sodium, potassium, magnesium, zinc, tin and/or lithium having a chain length of 6 to 20 C atoms in the straight-chain or branched, saturated, mono- or polyunsaturated fatty acid radicals.

8. Process according to Claim 1, **characterized in that** the reaction is carried out according to process step a) using an equimolar substance quantitative ratio of fatty acid alkyl ester to sucrose or an excess of fatty acid alkyl ester, in particular in a substance quantitative ratio of fatty acid alkyl ester to sucrose in the range from 1:1 to 3.5:1.

9. Process according to Claim 1, **characterized in that** the filtration according to process step c) is carried out at 80 to 100°C and at a pressure of 0.5 to 2 bar, optionally in the presence of 0.5 to 2% by weight of filter aid.

## Revendications

1. Procédé pour la préparation sans solvant d'esters de saccharose avec des acides gras, dans lequel
a) on fait réagir du saccharose avec des esters alkyliques d'acides gras ayant une longueur de chaîne de 6 à 20 atomes de carbone dans le reste acide gras à chaîne droite ou ramifié, saturé ou une ou plusieurs fois insaturé, et de 1 à 6 atomes de carbone dans le reste alcool à chaîne droite ou ramifié, en présence de catalyseurs basiques, sous une pression de 25 à 100 mbars et à une température de 120 à 160°C,
**caractérisé en ce que**
b) on fait réagir le mélange réactionnel selon a) avec 0,01 à 0,5 mole de polyol par mole d'ester alkylique d'acide gras utilisé, sous une pression de 25 à 100 mbars et à une température de 90 à 130°C , en l'espace de 0,5 à 3 heures, et ensuite
c) on le filtre sans addition de solvants.

2. Procédé selon la revendication 1, **caractérisé en ce qu'**on utilise en tant que polyol du glycérol.

3. Procédé selon la revendication 1, **caractérisé en ce qu'**on utilise des esters alkyliques d'acides gras ayant une longueur de chaîne de 12 à 18 atomes de carbone dans le reste acide gras.

4. Procédé selon la revendication 1, **caractérisé en ce qu'**on utilise des esters alkyliques d'acides gras ayant une longueur de chaîne de 1 à 6 atomes de carbone dans le reste alcool à chaîne droite ou ramifié.

5. Procédé selon la revendication 1, **caractérisé en ce** dans l'étape a) du procédé on utilise des esters méthyliques d'acides gras.

6. Procédé selon la revendication 1, **caractérisé en ce que** la réaction selon l'étape a) du procédé comprend la présence de 0,5 à 5 % en poids du catalyseur, par rapport au poids total du mélange réactionnel.

7. Procédé selon la revendication 6, **caractérisé en ce que** le catalyseur est choisi dans le groupe comprenant des hydroxydes de métaux alcalins, en particulier l'hydroxyde de sodium et l'hydroxyde de potassium, des carbonates de métaux alcalins, en particulier le carbonate de potassium et le carbonate de césium, des savons et des savons métalliques des acides gras avec le sodium, le potassium, le magnésium, le zinc, l'étain et/ou le lithium, ayant une longueur de chaîne de 6 à 20 atomes de carbone dans les restes acide gras à chaîne droite ou ramifiés, saturés ou une ou plusieurs fois insaturés.

8. Procédé selon la revendication 1, **caractérisé en ce que** la réaction selon l'étape a) du procédé est effectuée avec un rapport équimolaire des quantités d'ester alkylique d'acide gras au saccharose ou un excès d'ester alkylique d'acide gras, en particulier en un rapport des quantités d'ester alkylique d'acide gras au saccharose dans la plage de 1:1 à 3,5:1.

9. Procédé selon la revendication 1, **caractérisé en ce qu'**on effectue la filtration selon l'étape c) du procédé à une température de 80 à 100°C et sous une pression de 0,5 à 2 bars, éventuellement en présence de 0,5 à 2 % en poids d'adjuvant de filtration.
